(19)

**Europäisches Patentamt**
**European Patent Office**
**Office européen des brevets**

(11) **EP 4 702 952 A1**

(12) **EUROPEAN PATENT APPLICATION**

(43) Date of publication:
04.03.2026 Bulletin 2026/10

(21) Application number: 25199714.4

(22) Date of filing: 02.09.2025

(51) International Patent Classification (IPC):
*A61F 9/007* (2006.01)     *A61B 3/14* (2006.01)
*A61F 9/008* (2006.01)

(52) Cooperative Patent Classification (CPC):
A61F 9/008; A61B 3/14; A61F 9/0079

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 02.09.2024 CN 202411226952

(71) Applicant: ZHEJIANG BOYE BIOTECH LIMITED
Huzhou Zhejiang 313000 (CN)

(72) Inventors:
• YU, Ayong
  Huzhou, 313000 (CN)

• CHEN, Yiyuan
  Huzhou, 313000 (CN)
• CHEN, Jianning
  Huzhou, 313000 (CN)
• HE, Huanyao
  Huzhou, 313000 (CN)
• LIU, Qiuyuan
  313000 Huzhou, 313000 (CN)

(74) Representative: Mewburn Ellis LLP
Aurora Building
Counterslip
Bristol BS1 6BX (GB)

(54) **APPARATUS AND METHOD FOR DELIVERING THERAPEUTIC LIGHT OR DETECTION LIGHT TO EYE TISSUE OF PATIENT**

(57)     The present application discloses an apparatus and a method for delivering therapeutic light or detection light to an eye tissue of a patient. The apparatus comprises a light source device, an image capture device, an actuating device, and a controller. The controller is coupled with the light source device, the image capture device, and the actuating device and configured to: control the image capture device to collect at least two images of the eye of the patient from different angles; extract at least iris information of the patient based on the at least two images collected from the different angles; determine the eye state of the patient based on at least the iris information; and control the light source device to or not to deliver the therapeutic light or detection light to the eye tissue of the patient and/or control the actuating device to move the light source device, based on the eye state of the patient.

EP 4 702 952 A1

## Description

## Technical Field

The present invention relates to the field of ophthalmic treatment, and more particularly, to an apparatus and a method for delivering therapeutic light or detection light to an eye tissue of a patient.

## Background Art

Irradiating eyes of patients with distinct wavelengths of laser or other rays to treat ocular diseases in the patients is a common ophthalmic treatment method. Examples include LASIK surgery using excimer laser to reshape corneal curvature to correct myopia, hyperopia, and astigmatism; retinal laser photocoagulation that treats diabetic retinopathy or retinal tears by applying green or yellow lasers to coagulate retinal lesion areas; and YAG laser posterior capsulotomy using infrared laser to disrupt posterior capsular opacification post cataract surgery. In the above-listed ophthalmic treatment methods, in order to achieve better treatment in lesioned tissues or areas to be operated on while effectively avoiding damage to healthy tissues, it is usually necessary to determine and monitor the eye state of the patient (e.g., whether the therapeutic ray is properly aligned with the site to be treated in the eyeball) before or during treatment. However, since such a treatment method is usually accompanied by local anesthesia of the eye of the patient and the use of devices such as eyelid speculum, the determination of the eye state of the patient mainly focuses on limited factors such as whether there are micromovements in the eyeball of the patient.

In addition, there are also some circumstances in which the eyes of patients are treated with ray irradiation in a non-anesthetic state. For example, a China patent application with publication number CN 113924143 A discloses an apparatus for treating cataract by emitting light with a specific wavelength to an affected eye by an LED light source. The apparatus usually takes 20 minutes each time to treat the cataract in the patient by means of light energy, and the eye of the patient is in a non-anesthetic state throughout the course. In this case, affected by physiological or other pathological factors and diverted attention, the eye of the patient may exhibit conditions such as shaking, tremors, blinking, and closing, thereby influencing the final therapeutic effect or leading to damage to healthy tissues. In addition, due to the relatively long treatment time of the apparatus, the traditional way of emitting detection light to the target position of the eye and processing the light signal reflected therefrom to obtain target position information may lead to a risk of excessive absorption of light energy by the eye. In addition, since the detection light may interfere with the therapeutic light, the final therapeutic effect may not reach the expectation.

Therefore, it is necessary to modify the existing ophthalmic treatment apparatus and method to improve the ability to detect the eye state of a patient.

## Summary of the Invention

An object of the present application is to provide an apparatus and a method for delivering therapeutic light or detection light to an eye tissue of a patient, with an improved ability to detect the eye state of the patient, thereby avoiding damage to normal tissues of the patient while therapeutic and detection effects are better achieved.

In one aspect of the present application, there is provided an apparatus for delivering therapeutic light or detection light to an eye tissue of a patient, comprising: a light source device configured to produce the therapeutic light or detection light; an image capture device configured to collect an image of an eye of the patient; an actuating device configured to move at least the light source device; and a controller coupled with the light source device, the image capture device, and the actuating device and configured to: control the image capture device to collect at least two images of the eye of the patient from different angles; extract at least iris information of the patient based on the at least two images collected from the different angles; determine the eye state of the patient based on at least the iris information; and control the light source device to or not to deliver the therapeutic light or detection light to the eye tissue of the patient and/or control the actuating device to move the light source device, based on the eye state of the patient.

In another aspect of the present application, there is provided a method for delivering therapeutic light or detection light to an eye tissue of a patient, comprising: control an image capture device to collect at least two images of the eye of the patient from different angles; extract at least iris information of the patient based on the at least two images collected from the different angles; determine the eye state of the patient based on at least the iris information; and control a light source device to or not to deliver the therapeutic light or detection light to the eye tissue of the patient and/or control an actuating device to move the light source device, based on the eye state of the patient.

In a further aspect of the present application, there is provided a non-volatile computer-readable storage medium, characterized in that the non-volatile computer-readable storage medium stores a computer program, and the computer program, when executed by a processor, implements the above method for delivering therapeutic light or detection light to an eye tissue of a patient.

The above is a summary of the present application, in which details may be simplified, generalized, and omitted, so those skilled in the art should realize that this section is only illustrative by way of example and is not intended to limit the scope of the present application in any way. This summary section is not intended to identify key features or essential features of the claimed subject matter, nor is it intended to be used as an auxiliary means in determining the scope of the claimed subject matter.

**Brief Description of the Drawings**

The above and other features of the content of the present application will be more fully and clearly understood from the following Description and append Claims in combination with the Drawings. It can be understood that the Drawings only depict several embodiments of the present application, and therefore should not be considered as limiting the scope of the present application. The content of the present application will be explained more clearly and in detail with the Drawings.

FIG. 1 shows a block diagram of an apparatus for delivering therapeutic light or detection light to an eye tissue of a patient according to one embodiment of the present application.

FIGS. 2A to 2C respectively show a perspective view, a front view, and a top view of a treatment module according to one embodiment of the present application.

FIGS. 3A and 3B respectively show an eye image of a patient before and after image processing according to one embodiment of the present application.

FIGS. 4A to 4G show schematic diagrams of iris contour information extracted from an eye image of a patient according to one embodiment of the present application.

FIGS. 5A and 5B show schematic views of an eye of a patient in an open state according to one embodiment of the present application.

FIGS. 6A and 6B show schematic views of an eye of a patient in a squinting state according to one embodiment of the present application.

FIGS. 7A and 7B show schematic views of an eye of a patient in a closed state according to one embodiment of the present application.

FIG. 8 shows a diagram of the principle of determining the distance between the center of a light outlet of a light source device and a preset central position of the light outlet according to one embodiment of the present application.

FIG. 9 shows a flow chart of a method for delivering therapeutic light or detection light to an eye tissue of a patient according to one embodiment of the present application.

**Detailed Description of Embodiments**

In the following detailed description, reference is made to the Drawings that form a part hereof. In the Drawings, similar symbols generally indicate similar components unless the context indicates otherwise. The illustrative embodiments described in the detailed description, the Drawings, and the Claims are not intended to be limiting. Other embodiments may be adopted, and other changes may be made without departing from the essence or scope of the subject matter of the present application. It can be understood that various aspects of the content of the present application, which are generally described in the present application and illustrated in the Drawings, can be configured, substituted, combined and designed in various forms, and all of these clearly constitute a part of the present application.

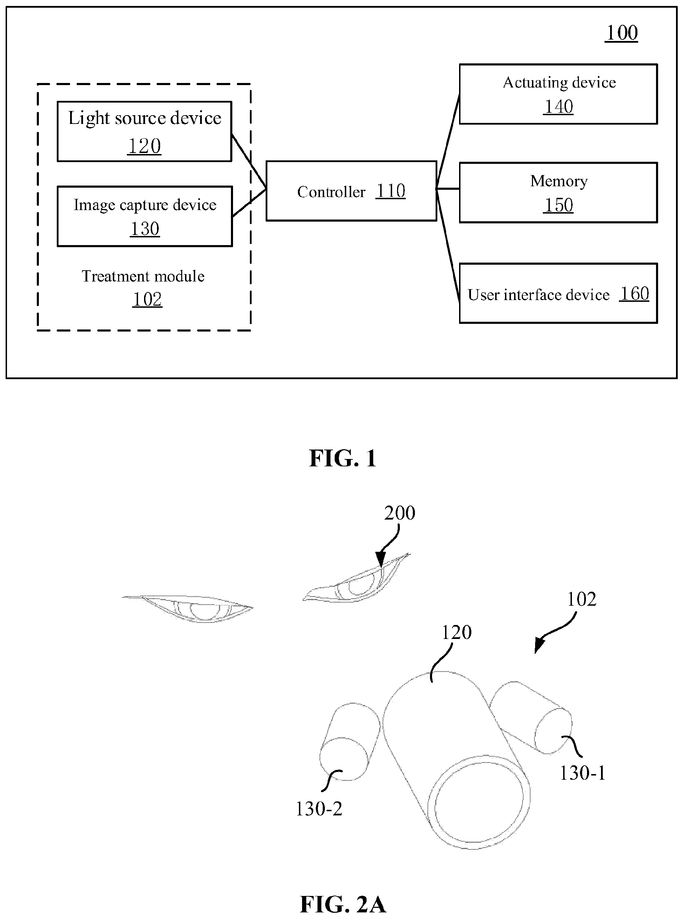

Referring to FIG. 1, it shows a block diagram of an ophthalmic treatment apparatus 100 for delivering therapeutic light or detection light to an eye tissue of a patient according to one embodiment of the present application. As shown in FIG. 1, the ophthalmic treatment apparatus 100 comprises a controller 110, and a light source device 120, an image capture device 130, an actuating device 140, a memory 150 and a user interface device 160 which are communicatively coupled to the controller 110.

The light source device 120 is configured to produce the therapeutic light or detection light. The light source device 120 may be either a laser light source or a non-laser light source, such as an LED light source. In some embodiments, the ophthalmic treatment apparatus 100 is configured to deliver therapeutic light with a preset wave band to a lens of a patient to treat cataract disease in the eye of the patient. In some embodiments, the light source device 120 is configured to emit therapeutic light with a wavelength ranging from 400 nm to 570 nm to the lens of the patient to treat the eye of the patient. In some embodiments, the wavelength of the therapeutic light of the light source device 120 ranges from 410 nm to 420 nm. In some embodiments, the light source device 120 is configured to send detection light to the eye tissue of the patient, for example, to emit light with a wavelength ranging from 350 nm to 410 nm, preferably ranging from 360 nm to 370 nm, more preferably light with a wavelength of 365 nm, to excite fluorescent light in a cataract. It can be understood that in different embodiments, the therapeutic light or detection light generated by the light source device 120 may have different wavelengths for different ocular diseases, and the present application is not limited to the above example wavelengths.

The image capture device 130 is configured to collect images of the eye of the patient, so that the controller 110 can determine the eye state of the patient based on these images. In the example of FIG. 1, the image capture device 130 and the light source device 120 are arranged together to form a treatment module 102 of the ophthalmic treatment apparatus 100. The actuating device 140 is configured to move at least the light source device 120. When the light source device 120 and the image capture device 130 are arranged together, the actuating device 140 can be mechanically coupled with the treatment module 102 to drive the treatment module 102 to move, so that the positions of the light source device 120 and the image capture device 130 relative to the eye of the patient can be adjusted simultaneously, thereby achieving alignment to a specific area in the eye of the patient. In some embodiments, the actuating device 140 may be a three-axis driving system that drives the treatment module 102 to move along the X-axis, Y-axis, and/or Z-axis. For example, the actuating device 140 may comprise three independent driving units (such as stepper motors or servo motors), so as to drive the treatment module 102 in three different dimensions. However, the present application is not limited to the above examples. In other embodiments, the light source device 120 and the image capture device 130 may be arranged separately, and the actuating device 140 may actuate the light source device 120 and the image capture device 130, separately.

The memory 150 is used for storing relevant data or program instructions of the ophthalmic treatment apparatus 100, comprising but not limited to a random access memory (RAM) for temporarily storing information, or a flash memory, hard disk, removable disk, read-only memory (ROM) for permanently storing information, etc. The user interface device 160 may comprise any device capable of interacting with a user, comprising but not limited to a display, a touch screen interface, a keyboard, an audio input or output device, a mouse, a joystick or buttons, etc. The controller 110 is coupled with the light source device 120, the image capture device 130, the actuating device 140, the memory 150, and/or the user interface device 160, and can execute programs stored in the memory 150 or receive instructions from the user interface device 160 to complete tasks or operations, for example, controlling or operating the light source device 120, the image capture device 130, the actuating device 140, the memory 150, and/or the user interface device 160 to deliver therapeutic light or detection light to the eye tissue of the patient according to a specific therapeutic regimen. The controller 110 may comprise a general-purpose or special-purpose processor, a controller, a field programmable gate array (FPGA), etc. In an embodiment of the present invention, the controller 110 can control the image capture device 130 to collect at least two images of the eye of the patient from different angles; extract at least iris information of the patient based on the at least two images collected from the different angles; determine the eye state of the patient based on at least the iris information; and control the light source device 120 to or not to deliver the therapeutic light or detection light to the eye tissue of the patient and/or control the actuating device 140 to move the light source device 120, based on the eye state of the patient.

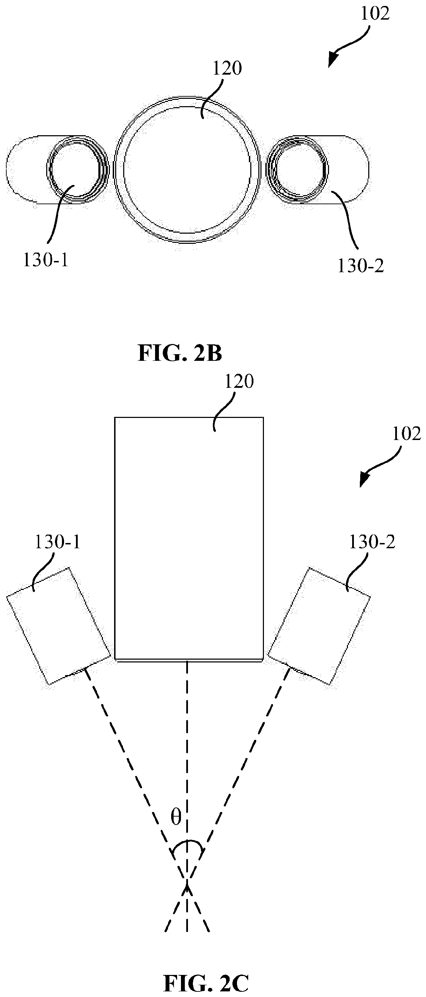

Referring to FIGS. 2A to 2C, they respectively show a perspective view, a front view, and a top view of a treatment module 102 according to one embodiment of the present application. In this embodiment, the image capture device 130 comprises a first camera 130-1 and a second camera 130-2. The first camera 130-1 and the second camera 130-2 are installed at preset angles with respect to the light source device 120, so that images of the eye 200 of the patient to be treated can be acquired from different angles. As shown in FIG. 2B, the first camera 130-1 and the second camera 130-2 are symmetrically arranged on two sides of the light outlet of the light source device 120 along the radial direction of the light source device 120. As shown in FIG. 2C, the optical axes of the first camera 130-1 and the second camera 130-2 intersect the axis of the light source device 120 at the same point, and the included angle θ between the optical axes of the first camera 130-1 and the second camera 130-2 is greater than 0 degrees and less than 120 degrees, preferably 40 to 80 degrees. In some embodiments, during treatment, the eye of the patient reflects light with a wavelength of 360 nm-530 nm when continuously receiving the therapeutic light or the detection light. In order to reduce the influence of the reflected light on the visual determination by the cameras during real-time monitoring, corresponding optical filters can also be arranged in the first camera 130-1 and the second camera 130-2.

It needs be noted that although the first camera 130-1 and the second camera 130-2 are arranged on the two sides of the light outlet of the light source device 120 in the embodiments of FIGS. 2A to 2C, the present application is not limited thereto, and in some other embodiments, the first camera 130-1 and the second camera 130-2 may also be arranged at other positions where eye images of the patient can be collected at different angles. In some further embodiments, the image capture device 130 may comprise three or more cameras to collect three or more images of the eye of the patient from different angles. For example, in addition to the first camera and the second camera arranged on the two sides of the light source device 120, the image capture device 130 may also comprise a third camera or more cameras arranged at other positions of the light source device 120 (e.g., coaxial with the light source device 120). In other further embodiments, the camera capture device 130 may only comprise one camera, and the controller 110 may control the camera to move, so as to collect eye images of the patient at different angles.

Hereinafter, the apparatus for delivering therapeutic light or detection light to an eye tissue of a patient according to the present application will be described in detail with reference to the ophthalmic treatment apparatus 100 shown in FIG. 1 and the treatment module 102 having two cameras as shown in FIGS. 2A to 2C. It can be understood that the technical solution of the present application is also suitable for the case of having one, three, or more cameras, and the ability to detect the eye state of the patient can also be improved after proper adjustment and deformation, so as to avoid damage to normal tissues in the eye of the patient while the therapeutic effect is better achieved.

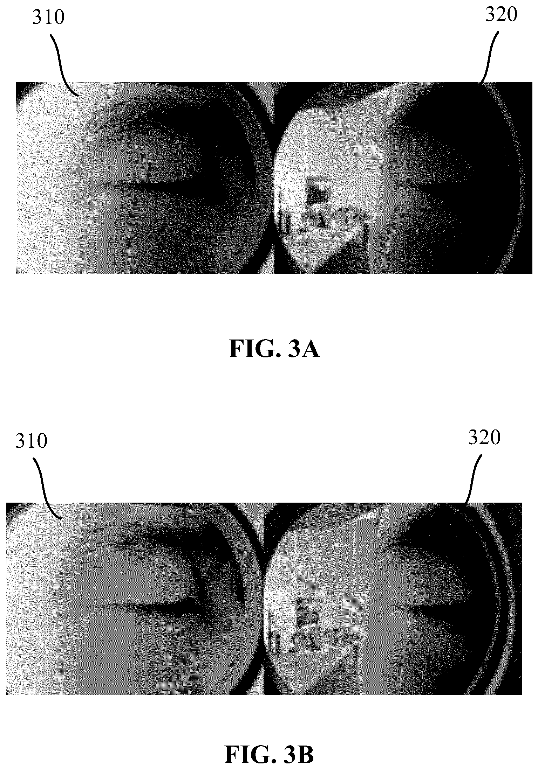

Referring to FIG. 3A, the controller 110 controls the first camera 130-1 to collect a first image 310 of the eye 200 of the patient from a first angle and controls the second camera 130-2 to collect a second image 320 of the eye 200 of the patient from a second angle different from the first angle. After receiving the first image 310 and the second image 320, the controller 110 extracts first eye information a1 and first iris information b1 from the first image 310 and extracts second eye information a2 and second iris information b2 from the second image 320.

In some embodiments, the first eye information a1 and the second eye information a2 can comprise whether eye feature information, eye contour information, and/or other eye information are extracted from the first image 310 and the second image 320. The first iris information b1 and the second iris information b2 may comprise whether feature information, contour information, shape information, gradient information, color information, texture information, etc. of the iris is extracted from the first image 310 and the second image 320. Specifically, since there are obvious color differences between the iris and surrounding eye tissues, these differences constitute an edge feature of the iris. During image processing, after preprocessing such as image enhancement, threshold segmentation and contour detection can be used to extract the edge contour of the iris and obtain the contour information of the iris. The overall shape of the iris may vary from person to person, but it is basically close to a circle, an ellipse, etc. During image processing, similarly after preprocessing such as image enhancement, a circle or arc in the image can be detected by using methods such as Hough circle detection, and then, a circle that fits the edge of the iris can be selected from a plurality of detected circles by setting a screening strategy, thereby obtaining the shape information of the iris. The gradient value reflects the pixel intensity change rate in the image. The gradation from the center to the edge of the iris and the sudden change at the edge are both useful information. For example, in an image, from each pixel, the gradient can be calculated, and the gradient contains the amplitude and direction. For the eye image, the closer to the center of the eyeball, the lower the gray value, and the more the connecting lines of gradient directions that cross at that point. The determination of the position of the center is to find the point at which the straightest lines of the gradient directions intersect, thus obtaining the iris center position information. The color information of the iris can include the basic color, color distribution, color intensity, and other information, among which the basic color is usually divided into several main categories such as blue, brown, and green; the color distribution refers to the distribution of colors on the iris, such as spots, streaks, or radial patterns; and the color intensity refers to the depth of a color, such as the distinction between dark brown and light brown. The fine texture of the iris surface can be captured from high-resolution images. In addition, although the distribution of blood vessels in the iris is not the main basis for iris recognition, it can also be used as an auxiliary feature in some cases; and the texture information of the iris is a common feature used to assist detection. Although some examples of the iris information are given above, the present application is not limited thereto. According to different application scenarios, the iris information can also comprise position information, size information, semantic information, etc.

The controller 110 extracts first eye information a1 and first iris information b1 from the first image 310 and extracts second eye information a2 and second iris information b2 from the second image 320, which can be performed either by an image processing method or by an algorithm based on machine learning or deep learning.

Hereinafter, the technical solution of the present application will be explained with eye information and iris information obtained by the image processing method.

In some embodiments, after the first image 310 and the second image 320 are acquired, the first image 310 and the second image 320 can be processed first, and then, the eye information and iris contour information can be extracted. For example, gamma correction and perspective transformation correction may be performed on the first image 310 and the second image 320.

As a specific example, the gamma correction process may comprise the following steps S11-S13. In step S11, the first image 310 and the second image 320 are respectively subjected to image grayscale conversion processing using a weighted averaging method to form grayscale images. In step S12, filtering processing is performed on each pixel in the grayscale images. For example, each pixel in the grayscale image is processed based on spatial proximity and pixel value similarity. In step S13, the grayscale image obtained after filtering processing is processed by an adaptive gamma correction method. For example, the grayscale image is filtered by a Gaussian filter to obtain a template, wherein the kernel of the Gaussian filter is more than 3*3 pixels; all the elements in the template are then subjected to scaling and nonlinear function mapping; the value of each element in the template processed by scaling and nonlinear function mapping is denoted as a gamma value; and based on the derived plurality of gamma values, the grayscale image obtained after filtering processing was subjected to gamma correction pixel by pixel. Referring to FIG. 3B, it shows the first image 310 and the second image 320, which have undergone gamma correction. It can be seen that by denoting the value of each element in the template processed by scaling and nonlinear function mapping as a gamma value, and based on the derived plurality of gamma values, subjecting the grayscale image obtained after filtering processing to gamma correction pixel by pixel, areas with low brightness in the image can be effectively enhanced while areas with high brightness are suppressed, thereby realizing a function of balancing the overall brightness of the image.

Since the first camera 130-1 and the second camera 130-2 are installed at non-perpendicular angles, perspective distortion may appear in the shot first image 310 and second image 320, that is, a distant object in the image looks smaller than a nearby one. In order to make the first image 310 and the second image 320 look like they were shot from the front side, perspective transformation can be applied to correct the images, so that the subsequent target detection can be more accurate. Perspective transformation can be used before or after any one of the above gamma correction steps S11-S13. As a specific example, perspective transformation correction may comprise the following steps S21-S23. In step S21, based on the installation angles of the first camera 130-1 and the second camera 130-2, the expected values of the four corner points of the image after perspective transformation are calculated. In step S22, using the four corner points of the image and the expected values of the four corner points obtained in step S21, a perspective transformation matrix is calculated by using a "get Perspective Transform" function in OpenCV library. In step S23, perspective transformation is carried out by using a "warp Perspective" function in OpenCV to output a corrected image. The input of the "warp Perspective" function comprises the original image, the perspective transformation matrix, and the expected output image size. The first image 310 and the second image 320 corrected by perspective transformation can better reflect the geometric relationship in the real world, which is helpful to improve the accuracy of calibration and three-dimensional coordinate calculation of the first camera 130-1 and the second camera 130-2.

The image processing of the first image 310 and the second image 320 is described above by taking gamma correction and perspective transformation correction as examples. It can be understood that the present application is not limited thereto. In some other embodiments, other processing methods can be used to process the first image 310 and the second image 320, as long as it is helpful to extract eye information and iris information.

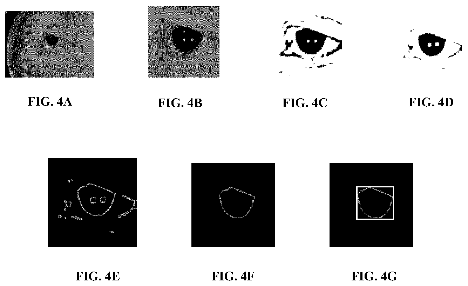

Hereinafter, with reference to FIGS. 4A to 4G, an example process of extracting first iris contour information b1 from the first image 310 or extracting second iris contour information b2 from the second image 320 is described.

Firstly, an eye region can be taken from the original image (as shown in FIG. 4A) of the first image 310 (or the second image 320), thereby obtaining an eye region image (as shown in FIG. 4B).

Next, a local threshold segmentation method is applied to the eye region image to segment the image into foreground and background, so that an image from local threshold segmentation is obtained (as shown in FIG. 4C). Specifically, the gray value of each pixel in the window is compared with a local threshold. If the gray value of the pixel is greater than the local threshold, the pixel is marked as foreground (target); and if the gray value of the pixel is less than or equal to the local threshold, the pixel is marked as background (non-target).

Next, the segmented image is subjected to morphological operations, such as erosion and dilation, to fill holes and connect adjacent segmented regions, etc., so as to remove noise and further refine the shape of the iris, thereby obtaining a morphologically processed image (as shown in FIG. 4D).

Next, a "findContours( )" function in OpenCV is used to find all contours in the image, and external contours are obtained by specifying "cv2.RETR_EXTERNAL", so that an image resulting from contour detection is obtained (as shown in FIG. 4E).

Still next, the contour in the image after contour detection also comprises the contours of the eyelid and some eyebrows, so it is necessary to set screening conditions (such as the area and length of the contour) to screen out the contour that is most likely to be the iris, thereby obtaining an image after contour filtration (as shown in FIG. 4F), thus obtaining first iris contour information b1 (or second iris contour information b2).

In addition, after the iris contour information b1 is extracted, the maximum circumscribed rectangle of the iris contour can be further obtained (as shown in FIG. 4G), and the center coordinates of the maximum circumscribed rectangle can be calculated as the coordinates of the iris center point, which can be used subsequently to determine the eye state of the patient. It should be noted that in the present application, the coordinates of the iris center point are taken as the coordinates of the center of the pupil to realize the delivery of the therapeutic light to the pupil.

In some embodiments, the extraction of first eye information a1 from the first image 310 or the extraction of second eye information a2 from the second image 320 can comprise the following steps. Firstly, an eye region is extracted from the first image 310 (or the second image 320) by Haar cascade classifier, and then, the eye region is cut out from the original image. Next, an image processing method in OpenCV is applied to further refine the extraction of eye features. Haar cascade is a feature-based object detection method, which realizes object detection by classifying features in the image in cascade. It uses Haar features as basic image features. These features can capture grayscale changes in different areas of the image, thus constructing a cascade classifier. This method involves first sliding a window with a specific size in the image, then calculating the values of a plurality of Haar features in each window, and finally determining whether these windows contain the target object by the cascade classifier. This method has been widely used in face detection and other fields due to its advantages in computational efficiency and accuracy.

After the first eye information a1 and the first iris contour information b1 are extracted from the first image 310 and the second eye information a2 and the second iris contour information b2 are extracted from the second image 320, the controller 110 can determine the eye state of the patient based on the first eye information a1 and the second eye information a2 and the first iris contour information b1 and the second iris contour information b2.

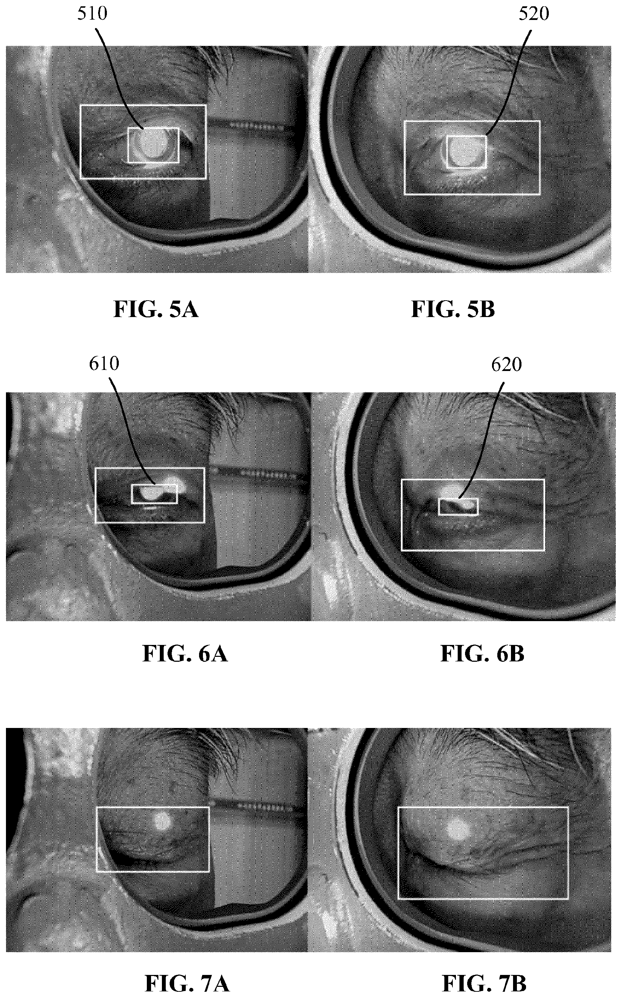

Specifically, referring to FIGS. 5A and 5B, when the first iris contour information b1 and the second iris contour information b2 indicate the presence of the iris contour and the height-to-width ratios of the first circumscribed rectangle 510 and the second circumscribed rectangle 520, which correspond to the first iris contour information b1 and the second iris contour information b2, respectively, are both not less than the preset numerical value, the controller 110 determines that the eye of the patient is in an open state. In some embodiments, the preset numerical value is between 0.2 to 0.6. Preferably, the preset numerical value is 0.5.

Referring to FIGS. 6A and 6B, when the first iris contour information b1 and the second iris contour information b2 indicate the presence of the iris contour and the height-to-width ratios of the first circumscribed rectangle 610 and the second circumscribed rectangle 620, which correspond to the first iris contour information b1 and the second iris contour information b2, respectively, are both less than the preset numerical value, the controller 110 determines that the eye of the patient is in a squinting state.

Referring to FIGS. 7A and 7B, when at least one of the first eye information a1 and the second eye information a2 indicates the presence of the eye and at least one of the first iris contour information b1 and the second iris contour information b2 indicates the absence of the iris contour, the controller 110 determines that the eye of the patient is in a closed state.

In addition, when the first iris contour information b1 and the second iris contour information b2 indicate the presence of an iris contour, the distance between the center of the light outlet of the light source device 120 and the preset central position of the light outlet is determined, and when the distance is greater than a preset threshold, the eye of the patient is determined to be in an improperly aligned state. When the distance is less than or equal to the preset threshold, the eye of the patient is determined to be in a properly aligned state.

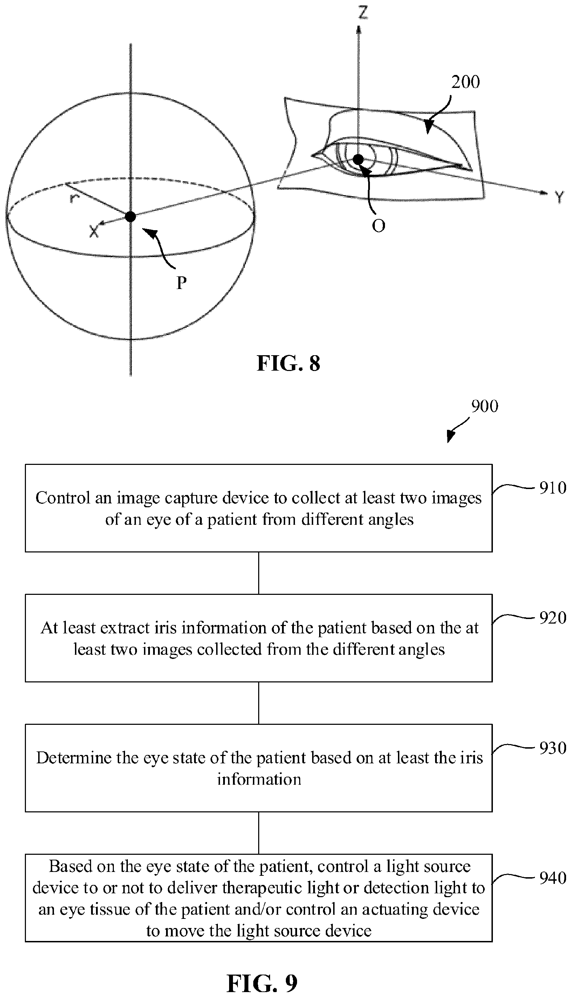

In some embodiments, the step of the controller 110 determining the distance between the center of the light outlet of the light source device 120 and the preset central position of the light outlet may comprise the following steps. Referring to FIG. 8, firstly, a three-dimensional coordinate system (X, Y, Z) is established with the iris center point of the eye of the patient as the origin O, and the preset central position P of the light outlet is located on the coordinate axis X of the three-dimensional coordinate system. The distance between the preset central position P of the light outlet and the origin O of the three-dimensional coordinate system is between 20 mm to 150 mm. Next, the coordinate values of the center of the light outlet of the light source device 120 in the three-dimensional coordinate system is determined. Since the relative positional relationships and distances between the first camera 130-1 and second camera 130-2 and the light source device 120 are determined in specific applications, the coordinate values of the center of the light outlet of the light source device 120 in the three-dimensional coordinate system can be determined by using a binocular ranging algorithm based on the images collected by the first camera 130-1 and the second camera 130-2 at different angles. Next, based on the coordinate values, the distance r between the center of the light outlet of the light source device 120 and the preset central position P of the light outlet is further calculated. The distance r can be expressed as

$$r = \sqrt{x^2 + y^2 + z^2}$$

, wherein x, y, and z are respectively the differences between the coordinates of the central point of the light outlet and the preset central position P of the light outlet in the three-dimensional coordinate system. When the distance r is greater than the preset threshold, the eye of the patient is determined to be in an improperly aligned state; and when the distance r is less than or equal to the preset threshold, the eye of the patient is determined to be in a properly aligned state. The preset threshold is between 0.1 mm and 1 mm, preferably 0.5 mm. With continued reference to FIG. 8, in the three-dimensional coordinate system (X, Y, Z), with the preset central position P of the light outlet as the spherical center, if the central point of the light outlet is in a sphere with P as the spherical center and a preset threshold value (e.g., 0.5 mm) as the radius (i.e., r < 0.5 mm), the eye of the patient is determined to be in the properly aligned state; otherwise, the eye of the patient is determined to be in the improperly aligned state. The actuating device (e.g., a three-axis driving system) moves based on the differences between the three-dimensional coordinates of the central point of the light outlet and the three-dimensional coordinates of the preset central position P of the light outlet, so when the first iris contour information b1 and the second iris contour information b2 are extracted, the purpose of eliminating the jitter of the three-axis driving system can be achieved by setting r < 0.5 mm to be the aligned state.

In some other embodiments, when the first eye information a1 and the first iris contour information b1 indicate that no eye feature information and no iris feature information are extracted from the first image 310, or the second eye information a2 and the second iris contour information b2 indicate that no eye feature information and no iris feature information are extracted from the second image 320, a state of the eye of the patient not being detected is determined.

The specific steps of obtaining eye information and iris information and determining the eye state of the patient by using the image processing method are described above with a specific example. However, the present application is not limited thereto. In other embodiments, other image processing methods may also be used to obtain eye information and iris information and/or determine the eye state of the patient according to specific needs.

For example, the iris center point can be determined based on gradient direction information, which comprises the steps of: a. Gradient calculation: an original image is subjected to grayscale conversion and edge detection to obtain the gradient value and direction of each pixel; b. Gradient direction analysis: in an iris image, the gradient directions of pixels near the iris center typically point toward the iris center because the grayscale change between the pupil edge and the iris is relatively significant, which leads to the gradient direction mostly towards the iris center; and c. Central point localization: all gradient directions are counted, and the point at which most of the gradient directions meet is found out, which is namely the iris center.

In some other examples, the iris center point can be determined based on Hough transformation, which can comprise the steps of: a. Edge detection: a method such as Canny edge detection is used to detect edges in the image; b. Hough circle transformation: Hough circle transformation is applied to detect a circular contour in the image, wherein Hough circle transformation can detect the circular object in the image effectively even if the boundary is incomplete; and c. Circle center localization: the circle center coordinates returned by Hough circle transformation are namely the estimated position of the iris center.

In some further examples, the iris center point can be determined based on threshold segmentation, which can comprise the steps of: a. Threshold segmentation: according to the gray value range of the image, an appropriate threshold is selected, and the image is divided into two parts, i.e., foreground (iris) and background (the rest); b. Morphological operations: morphological operations such as erosion and dilation are used to remove noise and refine contours; and c. Centroid calculation: the centroid of the segmented foreground (iris) is calculated, which is namely the iris center.

In some further examples, the iris center point can also be determined by combining various methods. For example, combining the results of various methods such as gradient direction, Hough transformation, and threshold segmentation, the final iris center position is determined by weighted average or other fusion strategies.

In the above embodiments, an embodiment in which the controller 110 uses an image processing method to obtain iris information and determine the eye state of the patient is described. However, the present application is not limited thereto. In some other embodiments, the controller 110 may also use machine learning or deep learning algorithms to extract iris information of the patient from the image and/or determine the eye state of the patient.

An iris detection algorithm based on machine learning or deep learning algorithm can use the powerful ability of models such as Convolutional Neural Networks (CNNs) to detect and locate the iris center in the image. Hereinafter, the input information, output information, and common models when using machine learning or deep learning algorithms to obtain iris information are introduced.

The input information may comprise an eye image. Specifically, the input information can be either a colored image or a black-and-white image, and the size can be adjusted according to the requirements of the model. For example, if the model requires an input size of $3 \times 640 \times 640$ pixels, the input image needs to be scaled to this size. Before input to the network, the image may also need to be preprocessed, e.g., by standardization or normalization, such as dividing by 255 to make the pixel value between 0 and 1, or subtracting the average and dividing the result by the standard deviation to standardize the image data.

The output information may comprise the coordinates of the iris center. The output information of the model is usually the center coordinates (x, y) and the height and width (h, w) of the iris circumscribed rectangle, wherein the (x, y) is a position in the input image in the coordinate system. The output information of the model can also comprise confidence scores. For example, the model may also output a confidence score, indicating the reliability of the model prediction result.

In some embodiments, models such as a target detection model and a semantic segmentation model can be used to detect and locate the iris center in the image. The target detection model is typically used to locate specific objects within the image and give the positional coordinates of these objects. Target detection models may include yolo series models (e.g., yolov3, yolov4, yolov5, ..., yolov8, yolov9, yolov10, and yolox), rcnn series models (e.g., Fast R-CNN, Faster R-CNN, and Mask R-CNN), and other detection models (e.g., SSD and CenterNet). The semantic segmentation model can divide the image into different regions and assign a category label to each pixel. For the task of iris detection, this model can be used to segment the iris region and locate the iris center by calculating the segmentation result. Semantic segmentation models can include U-Net, DeepLab, FCN, PSPNet, etc.

After the eye state (i.e., the open state, squinting state, closed state, improperly aligned state, or state of the eye of the patient not being detected) of the patient is obtained, the controller 110 may also control the light source device 120 to or not to deliver therapeutic light or detection light to the eye tissue of the patient and/or control the actuating device to move the light source device 120, based on the eye state of the patient.

Specifically, when the eye of the patient is determined to be in the open state, the controller 110 controls the light source device 120 to deliver therapeutic light or detection light. When the eye of the patient is determined to be in the squinting state, the controller 110 controls the light source device 120 to deliver therapeutic light, and outputs a first prompt message after the squinting state lasts for a first preset length of time (e.g., 10 seconds). When the eye of the patient is determined to be in the closed state, the light source device 120 is controlled to deliver therapeutic light, and outputs a second prompt message after the closed state lasts for a second preset length of time (e.g., 6 seconds). In addition, when the eye of the patient is determined to be in the improperly aligned state, the controller 110 controls the light source device 120 to deliver therapeutic light and controls the actuating device 140 to move the light source device 120. When in the state of the eye of the patient not being detected, if the state of the eye of the patient not being detected lasts for a third preset length of time (e.g., 6 seconds), the controller 110 controls the light source device 120 to stop delivering the therapeutic light or the detection light. Furthermore, in some examples, the controller 110 can also control the actuating device 140 to return to a preset point.

In some embodiments, when the eye of the patient is determined to be in the improperly aligned state, if the eye of the patient is in the properly aligned state by moving the light source device 120, the light source device 120 is continuously on for the delivery of the therapeutic light or detection light; and if the state of the light source device 120 being moved but improperly aligned lasts for another preset length of time (e.g., 6 seconds) or more, the controller 110 outputs a third prompt message and controls the light source device 120 to stop delivering the therapeutic light or detection light. In some embodiments, pictures taken by the first camera 130-1 and the second camera 130-2 during movement may show torn and delayed phenomena, which may cause errors in the calculation of three-dimensional coordinates, causing the actuating device 140 (e.g., a three-axis driving system) to swing and follow the eyeball seriously, which violates the purpose of eliminating jitter, so during the movement of the light source device 120, image sampling may not be carried out.

In some embodiments, the radiation power of the therapeutic light or detection light delivered in the open state, squinting state, closed state, and improperly aligned state, as described above, is the same, which is beneficial to simplify the operation of the light source device 120. In some other embodiments, the radiation power of the therapeutic light or detection light delivered in the open state, squinting state, closed state, and improperly aligned state, as described above, may also be different or vary. For example, when the squinting state or closed state of the patient is detected, the light source device 120 can be controlled in advance to deliver the therapeutic light or detection light at a reduced radiation power possibly because the patient does not adapt to the stimulation of the therapeutic light or detection light. When the patient has adapted to the therapeutic light or detection light after the squinting state or closed state of the patient lasts for a fourth preset length of time (e.g., 6 seconds), the light source device 120 can be controlled to restore the original radiation power to continue delivering the therapeutic light or the detection light. Also, for example, when the eye of the patient is determined to be in the squinting state or closed state, the radiation power of the light source device 120 can be controlled to change stepwise. Specifically, when the eye of the patient is determined to be in the squinting state or closed state, the radiation power of the light source device 120 is controlled to be reduced from a first preset power to a second preset power; and after the radiation power of the light source device 120 is reduced to the second preset power, if the controller 110 determines that the eye of the patient is still in the squinting state or closed state, the radiation power of the light source device 120 is controlled to be reduced from the second preset power to a third preset power.

Still for example, when the eye of the patient is determined to be in the open state, the radiation power of the light source device 120 may also be controlled to change stepwise. Specifically, when the eye of the patient is determined to be in the open state, the radiation power of the light source device 120 is controlled to be increased from the first preset power to a fourth preset power; and after the radiation power of the light source device 120 is increased to the fourth preset power, if the controller 110 determines that the eye of the patient is still in the open state, the radiation power of the light source device 120 is controlled to be increased from the fourth preset power to a fifth preset power.

Furthermore, the controller 110 can provide the above first, second, and third prompt messages to the user via the user interface device 160 to inform the user of the current working state of the ophthalmic treatment apparatus 100, for example, by providing text information via a screen, or providing sound information via an audio playback device. Specifically, after the eye of the patient is determined to be in the squinting state and lasts for a first preset length of time, the controller 110 outputs a first prompt message (e.g., "Eyelid occlusion, please adjust") via the user interface device 160 to alert the user to the information of eyelid occlusion. After the eye of the patient is determined to be in the closed state and lasts for a second preset length of time, a second prompt message (e.g., "Eyelid occlusion, please adjust") is output via the user interface device to alert the user to the information of eyelid occlusion. After the eye of the patient is determined to be in the improperly aligned state and lasts for a third preset length of time, a third prompt message (e.g., "Eye alignment is not complete, please adjust") is output via the user interface device to alert the user to the information of the alignment to the eye of the patient not being complete.

An embodiment of the present application provides a method for delivering therapeutic light or detection light to an eye tissue of a patient. In some embodiments, as shown in FIG. 9, a method 900 for delivering therapeutic light or detection light to an eye tissue of a patient comprises the following steps 910-940. In step 910, an image capture device is controlled to collect at least two images of the eye of the patient from different angles. In step 920, iris information of the patient is at least extracted based on the at least two images collected from the different angles. In step 930, the eye state of the patient is determined based on at least the iris information. In step 940, based on the eye state of the patient, a light source device is controlled to or not to deliver the therapeutic light or detection light to the eye tissue of the patient and/or an actuating device is controlled to move the light source device. The method 900 can be performed by the apparatus 100 as described above for delivering therapeutic light or detection light to an eye tissue of a patient. More details thereof can be referred to the above description in conjunction with FIGS. 1 to 8 and will not be repeated here. It should be noted that when the apparatus 100 is used to treat the eye of the patient, the above method 900 can be performed periodically many times, so that the state of the eye of the patient can be continuously monitored, and a corresponding treatment can be carried out.

An embodiment of the present application further provides a non-volatile computer-readable storage medium. The non-volatile computer-readable storage medium stores a computer program, and the computer program, when executed by a processor, executes the method for delivering therapeutic light or detection light to an eye tissue of a patient, as described in the above embodiment. In some embodiments, the non-volatile computer-readable storage medium can be a flash memory, a read-only memory (ROM), an electrically programmable ROM, an electrically erasable programmable ROM, a register, a hard disk, a removable disk, a CD-ROM, or any other form of non-volatile computer-readable storage medium commonly known in the technical field.

It should be noted that although several modules or sub-modules for the method and device for delivering therapeutic light or detection light to an eye tissue of a patient are mentioned in the above detailed description, this division is only exemplary and not mandatory. Actually, according to the embodiments of the present application, the features and functions of two or more modules described above can be embodied in one module. On the contrary, the features and functions of one module described above can be further divided into a plurality of modules and embodied thereby.

Those of ordinary skill in the art can understand and carry out other changes to the disclosed embodiments by studying the description, the disclosure, the Drawings, and the appended Claims. In the Claims, the word "comprising" does not exclude other elements and steps, and the words "a" and "an" do not exclude plural cases. In the practical application of the present application, one part may execute the functions of various technical features cited in the Claims. Any reference signs in the Claims shall not be construed as limiting the scope. The present disclosure includes the following numbered clauses:

Clause 1. An apparatus for delivering therapeutic light or detection light to an eye tissue of a patient, characterized by comprising:

a light source device configured to produce the therapeutic light or detection light;
an image capture device configured to collect an image of an eye of the patient;
an actuating device configured to move at least the light source device; and
a controller coupled with the light source device, the image capture device, and the actuating device and configured to:

control the image capture device to collect at least two images of the eye of the patient from different angles;
extract at least iris information of the patient based on the at least two images collected from the different angles;
determine the eye state of the patient based on at least the iris information; and
control the light source device to or not to deliver the therapeutic light or detection light to the eye tissue of the patient and/or control the actuating device to move the light source device, based on the eye state of the patient.

Clause 2. The apparatus according to clause 1, characterized in that the eye tissue is a lens, the therapeutic light has a preset wave band, and the apparatus is configured to deliver the therapeutic light to the lens of the patient to treat cataract disease.

Clause 3. The apparatus according to clause 1, characterized in that the iris information includes contour information, shape information, gradient information, color information, or texture information.

Clause 4. The apparatus according to clause 1, characterized in that the image capture device comprises a first camera and a second camera, wherein the first camera and the second camera are installed at preset angles relative to the light source device, and the step of the controller controlling the image capture device to collect at least two images of the eye of the patient from the different angles comprises:

controlling the first camera to collect a first image of the eye of the patient from a first angle; and
controlling the second camera to collect a second image of the eye of the patient from a second angle different from the first angle.

Clause 5. The apparatus according to clause 4, characterized in that the first camera and the second camera are installed on two sides of a light outlet of the light source device, and the included angle between optical axes of the first camera and the second camera is more than 0 degrees and less than 120 degrees.

Clause 6. The apparatus according to clause 1, characterized in that the step of the controller extracting at least the iris information of the patient based on the at least two images collected from the different angles comprises:

extracting first eye information and first iris contour information from the first image of the at least two images collected from the different angles; and
extracting second eye information and second iris contour information from the second image of the at least two images collected from the different angles.

Clause 7. The apparatus according to clause 6, characterized in that the step of the controller determining the eye state of the patient based on at least the iris information comprises:

when the first iris contour information and the second iris contour information indicate the presence of an iris contour and the height-to-width ratios of a first circumscribed rectangle and a second circumscribed rectangle corresponding to the first iris contour information and the second iris contour information, respectively, are both not less than a preset numerical value, determining that the eye of the patient is in an open state;
when the first iris contour information and the second iris contour information indicate the presence of the iris contour and the height-to-width ratios of a first circumscribed rectangle and a second circumscribed rectangle corresponding to the first iris contour information and the second iris contour information, respectively, are both less than the preset numerical value, determining that the eye of the patient is in a squinting state;
when at least one of the first eye information and the second eye information indicates the presence of the eye and at least one of the first iris contour information and the second iris contour information indicates the absence of the iris contour, determining that the eye of the patient is in a closed state;
when the first iris contour information and the second iris contour information indicate the absence of the iris contour, determining the distance between a center of the light outlet of the light source device and a preset central position of the light outlet, and when the distance is greater than a preset threshold, determining that the eye of the patient is in an improperly aligned state; or
when the first eye information and the first iris contour information indicate that no eye feature information and no iris feature information are extracted from the first image, or the second eye information and the second iris contour information indicate that no eye feature information and no iris feature information are extracted from the second image, determining a state of the eye of the patient not being detected.

Clause 8. The apparatus according to clause 7, characterized in that the preset numerical value is between 0.2 to 0.6.

Clause 9. The apparatus according to clause 7, characterized in that the step of the controller determining the distance between the center of the light outlet of the light source device and the preset central position of the light outlet comprises:

establishing a three-dimensional coordinate system with an iris center point of the eye of the patient as an origin, and the preset central position of the light outlet is located on a coordinate axis of the three-dimensional coordinate system;
determining the coordinate values of the center of the light outlet of the light source device in the three-dimensional coordinate system; and
based on the coordinate values, calculating the distance between the center of the light outlet of the light source device and the preset central position of the light outlet.

Clause 10. The apparatus according to clause 9, characterized in that the distance between the preset central position of the light outlet and the origin of the three-dimensional coordinate system is between 20 mm to 100 mm, and the preset threshold is between 0.1 mm to 1 mm.

Clause 11. The apparatus according to clause 7, characterized in that the step of the controller controlling the light source device to or not to deliver the therapeutic light or detection light to the eye tissue of the patient and/or controlling the actuating device to move the light source device, based on the eye state of the patient, comprises:

when the eye of the patient is determined to be in the open state, controlling the light source device to deliver the therapeutic light or the detection light;
when the eye of the patient is determined to be in the squinting state, controlling the light source device to deliver the therapeutic light, and after the squinting state lasts for a first preset length of time, outputting a first prompt message;
when the eye of the patient is determined to be in the closed state, controlling the light source device to deliver the therapeutic light, and after the closed state lasts for a second preset length of time, outputting a second prompt message;

when the eye of the patient is determined to be in the improperly aligned state, controlling the light source device to deliver the therapeutic light, and controlling the actuating device to move the light source device; or

when in the state of the eye of the patient not being detected, if the state of the eye of the patient not being detected lasts for a third preset length of time, controlling the light source device to stop delivering the therapeutic light or the detection light.

Clause 12. The apparatus according to clause 1, characterized in that the step of the controller controlling the light source device to or not to deliver the therapeutic light or detection light to the eye tissue of the patient, based on the eye state of the patient, comprises:

when the eye of the patient is determined to be in the squinting state or closed state, controlling the light source device to deliver the therapeutic light at a reduced radiation power; and
after the squinting state or the closed state lasts for a fourth preset length of time, controlling the light source device to restore the original radiation power and continue delivering the therapeutic light.

Clause 13. The apparatus according to clause 1, characterized in that the step of the controller controlling the light source device to or not to deliver the therapeutic light or detection light to the eye tissue of the patient, based on the eye state of the patient, comprises:

when the eye of the patient is determined to be in the squinting state or closed state, controlling the radiation power of the light source device to be reduced from a first preset power to a second preset power; and
after the radiation power of the light source device is reduced to the second preset power, if the controller determines that the eye of the patient is still in the squinting state or closed state, controlling the radiation power of the light source device to be reduced from the second preset power to a third preset power.

Clause 14. The apparatus according to clause 1, characterized in that the step of the controller controlling the light source device to or not to deliver the therapeutic light or detection light to the eye tissue of the patient, based on the eye state of the patient, comprises:

when the eye of the patient is determined to be in the open state, controlling the radiation power of the light source device to be increased from the first preset power to a fourth preset power; and
after the radiation power of the light source device is increased to the fourth preset power, if the controller determines that the eye of the patient is still in the open state, controlling the radiation power of the light source device to be increased from the fourth preset power to a fifth preset power.

Clause 15. The apparatus according to clause 11, characterized in that the apparatus further comprises a user interface device, wherein the controller outputs the first prompt message and the second prompt message to the user via the user interface device, the first prompt message and the second prompt message being configured to alert the user to eyelid occlusion.

Clause 16. A method for delivering therapeutic light or detection light to an eye tissue of a patient, characterized by comprising:

control an image capture device to collect at least two images of the eye of the patient from different angles;
extract at least iris information of the patient based on the at least two images collected from the different angles;
determine the eye state of the patient based on at least the iris information; and
control a light source device to or not to deliver the therapeutic light or detection light to the eye tissue of the patient and/or control an actuating device to move the light source device, based on the eye state of the patient.

Clause 17. The method according to clause 16, characterized in that the eye tissue is a lens, the therapeutic light has a preset wave band, and the method is used for delivering the therapeutic light to the lens of the patient to treat cataract disease.

Clause 18. The method according to clause 16, characterized in that the iris information includes contour information, shape information, gradient information, color information, or texture information.

Clause 19. The method according to clause 16, characterized in that the step of extracting at least the iris information of the patient based on the at least two images collected from the different angles comprises:

extracting first eye information and first iris contour information from the first image of the at least two images collected from the different angles; and
extracting second eye information and second iris contour information from the second image of the at least two images collected from the different angles.

Clause 20. The method according to clause 19, characterized in that the step of determining the eye state of the patient based on at least the iris information comprises:

when the first iris contour information and the second iris contour information indicate the presence of an iris contour and the height-to-width ratios of a first circumscribed rectangle and a second circumscribed rectangle corresponding to the first iris contour information and the second iris contour information, respectively, are both not less than a preset numerical value, determining that the eye of the patient is in an open state;

when the first iris contour information and the second iris contour information indicate the presence of the iris contour and the height-to-width ratios of a first circumscribed rectangle and a second circumscribed rectangle corresponding to the first iris contour information and the second iris contour information, respectively, are both less than the preset numerical value, determining that the eye of the patient is in a squinting state;

when at least one of the first eye information and the second eye information indicates the presence of the eye and at least one of the first iris contour information and the second iris contour information indicates the absence of the iris contour, determining that the eye of the patient is in a closed state;

when the first iris contour information and the second iris contour information indicate the absence of the iris contour, determining the distance between a center of the light outlet of the light source device and a preset central position of the light outlet, and when the distance is greater than a preset threshold, determining that the eye of the patient is in an improperly aligned state; or

when the first eye information and the first iris contour information indicate that no eye feature information and no iris feature information are extracted from the first image, or the second eye information and the second iris contour information indicate that no eye feature information and no iris feature information are extracted from the second image, determining a state of the eye of the patient not being detected.

Clause 21. The method according to clause 20, characterized in that the step of determining the distance between the center of the light outlet of the light source device and the preset central position of the light outlet comprises:

establishing a three-dimensional coordinate system with an iris center point of the eye of the patient as an origin, and the preset central position of the light outlet is located on a coordinate axis of the three-dimensional coordinate system;

determining the coordinate values of the center of the light outlet of the light source device in the three-dimensional coordinate system; and

based on the coordinate values, calculating the distance between the center of the light outlet of the light source device and the preset central position of the light outlet.

Clause 22. The method according to clause 20, characterized in that the step of controlling the light source device to or not to deliver the therapeutic light or detection light to the eye tissue of the patient and/or controlling the actuating device to move the light source device, based on the eye state of the patient, comprises:

when the eye of the patient is determined to be in the open state, controlling the light source device to deliver the therapeutic light or the detection light;

when the eye of the patient is determined to be in the squinting state, controlling the light source device to deliver the therapeutic light, and after the squinting state lasts for a first preset length of time, outputting a first prompt message;

when the eye of the patient is determined to be in the closed state, controlling the light source device to deliver the therapeutic light, and after the closed state lasts for a second preset length of time, outputting a second prompt message;

when the eye of the patient is determined to be in the improperly aligned state, controlling the light source device to deliver the therapeutic light, and controlling the actuating device to move the light source device; or

when in the state of the eye of the patient not being detected, if the state of the eye of the patient not being detected lasts for a third preset length of time, controlling the light source device to stop delivering the therapeutic light or the detection light.

Clause 23. The method according to clause 16, characterized in that the step of controlling the light source device to or not to deliver the therapeutic light or detection light to the eye tissue of the patient, based on the eye state of the patient, comprises:

when the eye of the patient is determined to be in the squinting state or closed state, controlling the light source device to deliver the therapeutic light at a reduced radiation power; and

after the squinting state or the closed state lasts for a fourth preset length of time, controlling the light source device to restore the original radiation power and continue delivering the therapeutic light.

Clause 24. The method according to clause 16, characterized in that the step of controlling the light source device to or not to deliver the therapeutic light or detection light to the eye tissue of the patient, based on the eye state of the patient, comprises:

when the eye of the patient is determined to be in the squinting state or closed state, controlling the radiation power of the light source device to be reduced from a first preset power to a second preset power; and
after the radiation power of the light source device is reduced to the second preset power, if the eye of the patient is still in the squinting state or closed state, controlling the radiation power of the light source device to be reduced from the second preset power to a third preset power.

Clause 25. The method according to clause 16, characterized in that the step of controlling the light source device to or not to deliver the therapeutic light or detection light to the eye tissue of the patient, based on the eye state of the patient, comprises:

when the eye of the patient is determined to be in the open state, controlling the radiation power of the light source device to be increased from the first preset power to a fourth preset power; and
after the radiation power of the light source device is increased to the fourth preset power, if the eye of the patient is still in the open state, controlling the radiation power of the light source device to be increased from the fourth preset power to a fifth preset power.

Clause 26. A non-volatile computer-readable storage medium, characterized in that the non-volatile computer-readable storage medium stores a computer program, and the computer program, when executed by a processor, implements the method for delivering therapeutic light to an eye tissue of a patient according to any one of clauses 16-25.

**Claims**

1. An apparatus for delivering therapeutic light or detection light to an eye tissue of a patient, **characterized by** comprising:

a light source device configured to produce the therapeutic light or detection light;
an image capture device configured to collect an image of an eye of the patient;
an actuating device configured to move at least the light source device; and
a controller coupled with the light source device, the image capture device, and the actuating device and configured to:

control the image capture device to collect at least two images of the eye of the patient from different angles;
extract at least iris information of the patient based on the at least two images collected from the different angles;
determine the eye state of the patient based on at least the iris information; and
control the light source device to or not to deliver the therapeutic light or detection light to the eye tissue of the patient and/or control the actuating device to move the light source device, based on the eye state of the patient.

2. The apparatus according to claim 1, **characterized in that** the eye tissue is a lens, the therapeutic light has a preset wave band, and the apparatus is configured to deliver the therapeutic light to the lens of the patient to treat cataract disease.

3. The apparatus according to claim 1 or 2, **characterized in that** the iris information includes contour information, shape information, gradient information, color information, or texture information.

4. The apparatus according to any one of the preceding claims, **characterized in that** the image capture device comprises a first camera and a second camera, wherein the first camera and the second camera are installed at preset angles relative to the light source device, and the step of the controller controlling the image capture device to collect at least two images of the eye of the patient from the different angles comprises:

controlling the first camera to collect a first image of the eye of the patient from a first angle; and
controlling the second camera to collect a second image of the eye of the patient from a second angle different from the first angle; and
optionally, the apparatus is **characterized in that** the first camera and the second camera are installed on two sides of a light outlet of the light source device, and the included angle between optical axes of the first camera and the second camera is more than 0 degrees and less than 120 degrees.

**5.** The apparatus according to any one of the preceding claims, **characterized in that** the step of the controller extracting at least the iris information of the patient based on the at least two images collected from the different angles comprises:

extracting first eye information and first iris contour information from the first image of the at least two images collected from the different angles; and
extracting second eye information and second iris contour information from the second image of the at least two images collected from the different angles.

**6.** The apparatus according to claim 5, **characterized in that** the step of the controller determining the eye state of the patient based on at least the iris information comprises:

when the first iris contour information and the second iris contour information indicate the presence of an iris contour and the height-to-width ratios of a first circumscribed rectangle and a second circumscribed rectangle corresponding to the first iris contour information and the second iris contour information, respectively, are both not less than a preset numerical value, determining that the eye of the patient is in an open state;
when the first iris contour information and the second iris contour information indicate the presence of the iris contour and the height-to-width ratios of a first circumscribed rectangle and a second circumscribed rectangle corresponding to the first iris contour information and the second iris contour information, respectively, are both less than the preset numerical value, determining that the eye of the patient is in a squinting state;
when at least one of the first eye information and the second eye information indicates the presence of the eye and at least one of the first iris contour information and the second iris contour information indicates the absence of the iris contour, determining that the eye of the patient is in a closed state;
when the first iris contour information and the second iris contour information indicate the absence of the iris contour, determining the distance between a center of the light outlet of the light source device and a preset central position of the light outlet, and when the distance is greater than a preset threshold, determining that the eye of the patient is in an improperly aligned state; or
when the first eye information and the first iris contour information indicate that no eye feature information and no iris feature information are extracted from the first image, or the second eye information and the second iris contour information indicate that no eye feature information and no iris feature information are extracted from the second image, determining a state of the eye of the patient not being detected; and
optionally, the apparatus is **characterized in that** the preset numerical value is between 0.2 to 0.6.

**7.** The apparatus according to claim 6, **characterized in that** the step of the controller determining the distance between the center of the light outlet of the light source device and the preset central position of the light outlet comprises:

establishing a three-dimensional coordinate system with an iris center point of the eye of the patient as an origin, and the preset central position of the light outlet is located on a coordinate axis of the three-dimensional coordinate system;
determining the coordinate values of the center of the light outlet of the light source device in the three-dimensional coordinate system; and
based on the coordinate values, calculating the distance between the center of the light outlet of the light source device and the preset central position of the light outlet; and optionally, the apparatus is **characterized in that** the distance between the preset central position of the light outlet and the origin of the three-dimensional coordinate system is between 20 mm to 100 mm, and the preset threshold is between 0.1 mm to 1 mm.

**8.** The apparatus according to claim 6 or 7, **characterized in that** the step of the controller controlling the light source device to or not to deliver the therapeutic light or detection light to the eye tissue of the patient and/or controlling the actuating device to move the light source device, based on the eye state of the patient, comprises:

when the eye of the patient is determined to be in the open state, controlling the light source device to deliver the therapeutic light or the detection light;
when the eye of the patient is determined to be in the squinting state, controlling the light source device to deliver the therapeutic light, and after the squinting state lasts for a first preset length of time, outputting a first prompt message;
when the eye of the patient is determined to be in the closed state, controlling the light source device to deliver the therapeutic light, and after the closed state lasts for a second preset length of time, outputting a second prompt message; when the eye of the patient is determined to be in the improperly aligned state, controlling the light source device to deliver the therapeutic light, and controlling the actuating device to move the light source device; or

when in the state of the eye of the patient not being detected, if the state of the eye of the patient not being detected lasts for a third preset length of time, controlling the light source device to stop delivering the therapeutic light or the detection light; and

optionally, the apparatus is **characterized in that** the apparatus further comprises a user interface device, wherein the controller outputs the first prompt message and the second prompt message to the user via the user interface device, the first prompt message and the second prompt message being configured to alert the user to eyelid occlusion.

9. The apparatus according to any one of the preceding claims, **characterized in that** the step of the controller controlling the light source device to or not to deliver the therapeutic light or detection light to the eye tissue of the patient, based on the eye state of the patient, comprises:

when the eye of the patient is determined to be in the squinting state or closed state, controlling the light source device to deliver the therapeutic light at a reduced radiation power; and after the squinting state or the closed state lasts for a fourth preset length of time, controlling the light source device to restore the original radiation power and continue delivering the therapeutic light.

10. The apparatus according to any one of the preceding claims, **characterized in that** the step of the controller controlling the light source device to or not to deliver the therapeutic light or detection light to the eye tissue of the patient, based on the eye state of the patient, comprises:

when the eye of the patient is determined to be in the squinting state or closed state, controlling the radiation power of the light source device to be reduced from a first preset power to a second preset power; and after the radiation power of the light source device is reduced to the second preset power, if the controller determines that the eye of the patient is still in the squinting state or closed state, controlling the radiation power of the light source device to be reduced from the second preset power to a third preset power.

11. The apparatus according to any one of the preceding claims, **characterized in that** the step of the controller controlling the light source device to or not to deliver the therapeutic light or detection light to the eye tissue of the patient, based on the eye state of the patient, comprises:

when the eye of the patient is determined to be in the open state, controlling the radiation power of the light source device to be increased from the first preset power to a fourth preset power; and after the radiation power of the light source device is increased to the fourth preset power, if the controller determines that the eye of the patient is still in the open state, controlling the radiation power of the light source device to be increased from the fourth preset power to a fifth preset power.

12. A non-volatile computer-readable storage medium, **characterized in that** the non-volatile computer-readable storage medium stores a computer program, and the computer program, when executed by a processor, implements a method for delivering therapeutic light or detection light to an eye tissue of a patient, **characterized in that** the method comprises:

control an image capture device to collect at least two images of the eye of the patient from different angles; extract at least iris information of the patient based on the at least two images collected from the different angles; determine the eye state of the patient based on at least the iris information; and control a light source device to or not to deliver the therapeutic light or detection light to the eye tissue of the patient and/or control an actuating device to move the light source device, based on the eye state of the patient.

13. The non-volatile computer-readable storage medium according to claim 12, **characterized in that** the step of extracting at least the iris information of the patient based on the at least two images collected from the different angles comprises:

extracting first eye information and first iris contour information from the first image of the at least two images collected from the different angles; and extracting second eye information and second iris contour information from the second image of the at least two images collected from the different angles.

14. The non-volatile computer-readable storage medium according to claim 13, **characterized in that** the step of determining the eye state of the patient based on at least the iris information comprises:

when the first iris contour information and the second iris contour information indicate the presence of an iris contour and the height-to-width ratios of a first circumscribed rectangle and a second circumscribed rectangle corresponding to the first iris contour information and the second iris contour information, respectively, are both not less than a preset numerical value, determining that the eye of the patient is in an open state;

when the first iris contour information and the second iris contour information indicate the presence of the iris contour and the height-to-width ratios of a first circumscribed rectangle and a second circumscribed rectangle corresponding to the first iris contour information and the second iris contour information, respectively, are both less than the preset numerical value, determining that the eye of the patient is in a squinting state;

when at least one of the first eye information and the second eye information indicates the presence of the eye and at least one of the first iris contour information and the second iris contour information indicates the absence of the iris contour, determining that the eye of the patient is in a closed state;

when the first iris contour information and the second iris contour information indicate the absence of the iris contour, determining the distance between a center of the light outlet of the light source device and a preset central position of the light outlet, and when the distance is greater than a preset threshold, determining that the eye of the patient is in an improperly aligned state; or

when the first eye information and the first iris contour information indicate that no eye feature information and no iris feature information are extracted from the first image, or the second eye information and the second iris contour information indicate that no eye feature information and no iris feature information are extracted from the second image, determining a state of the eye of the patient not being detected.

15. The non-volatile computer-readable storage medium according to claim 14, **characterized in that** the step of determining the distance between the center of the light outlet of the light source device and the preset central position of the light outlet comprises:

establishing a three-dimensional coordinate system with an iris center point of the eye of the patient as an origin, and the preset central position of the light outlet is located on a coordinate axis of the three-dimensional coordinate system;

determining the coordinate values of the center of the light outlet of the light source device in the three-dimensional coordinate system; and

based on the coordinate values, calculating the distance between the center of the light outlet of the light source device and the preset central position of the light outlet.

**FIG. 1**

**FIG. 2A**

102

120

130-1

130-2

**FIG. 2B**

120

102

130-1

130-2

θ

**FIG. 2C**

310 320

**FIG. 3A**

310 320

**FIG. 3B**

FIG. 4A

FIG. 4B

FIG. 4C

FIG. 4D

FIG. 4E

FIG. 4F

FIG. 4G

FIG. 5A          FIG. 5B

FIG. 6A          FIG. 6B

FIG. 7A          FIG. 7B

**FIG. 8**

Control an image capture device to collect at least two images of an eye of a patient from different angles — 910

At least extract iris information of the patient based on the at least two images collected from the different angles — 920

Determine the eye state of the patient based on at least the iris information — 930

Based on the eye state of the patient, control a light source device to or not to deliver therapeutic light or detection light to an eye tissue of the patient and/or control an actuating device to move the light source device — 940

**FIG. 9**

## EUROPEAN SEARCH REPORT

**Application Number**

EP 25 19 9714

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/124139 A1 (UNIV CALIFORNIA [US]; GENTRY THOMAS [US]; MORTON RYAN [US]) 13 June 2024 (2024-06-13) * abstract * * figure 4A * * page 21, line 11 - line 25 * * page 25, line 25 - page 26, line 22 * * page 63, line 1 - line 23 * * page 77, line 6 - line 21 * * page 78, line 10 - line 29 * * page 97, line 23 - line 28 * * page 98, line 8 - line 25 * ----- | 1-3,12 | INV. A61F9/007 A61B3/14 A61F9/008 |
| X | US 2009/161827 A1 (GERTNER MICHAEL [US] ET AL) 25 June 2009 (2009-06-25) * paragraphs [0041], [0152], [0160], [0177], [0179], [0180], [0197], [0219], [0240] * * figures 3A,5 * ----- | 1,3,4,12 | |
| A | US 2012/310083 A1 (FRIEDMAN MARC D [US] ET AL) 6 December 2012 (2012-12-06) * figure 10B * * paragraphs [0110], [0111] * ----- | 1-15 | TECHNICAL FIELDS SEARCHED (IPC) A61F A61B |
| A | US 2021/113373 A1 (SACKS ZACHARY [IL] ET AL) 22 April 2021 (2021-04-22) * the whole document * ----- | 1-15 | |

The present search report has been drawn up for all claims

| Place of search | Date of completion of the search | Examiner |
|---|---|---|
| Munich | 16 January 2026 | Jansen, Birte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 9714

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2026

| Patent document cited in search report | Publication date | Patent family member(s) | Publication date |
|---|---|---|---|
| WO 2024124139 A1 | 13-06-2024 | AU 2023390553 A1 | 19-06-2025 |
| | | CN 120529859 A | 22-08-2025 |
| | | EP 4611606 A1 | 10-09-2025 |
| | | KR 20250134597 A | 11-09-2025 |
| | | WO 2024124139 A1 | 13-06-2024 |
| US 2009161827 A1 | 25-06-2009 | CN 101951990 A | 19-01-2011 |
| | | EP 2231277 A2 | 29-09-2010 |
| | | EP 3272395 A1 | 24-01-2018 |
| | | US 2009161827 A1 | 25-06-2009 |
| | | US 2011081000 A1 | 07-04-2011 |
| | | US 2013315374 A1 | 28-11-2013 |
| | | WO 2009085204 A2 | 09-07-2009 |
| US 2012310083 A1 | 06-12-2012 | EP 2713849 A2 | 09-04-2014 |
| | | JP 6122845 B2 | 26-04-2017 |
| | | JP 2014519914 A | 21-08-2014 |
| | | US 2012310083 A1 | 06-12-2012 |
| | | US 2015265762 A1 | 24-09-2015 |
| | | WO 2012167260 A2 | 06-12-2012 |
| US 2021113373 A1 | 22-04-2021 | AU 2019297135 A1 | 19-11-2020 |
| | | AU 2022211843 A1 | 25-08-2022 |
| | | AU 2024205587 B2 | 08-01-2026 |
| | | CN 112351756 A | 09-02-2021 |
| | | CN 115804914 A | 17-03-2023 |
| | | EP 3817698 A1 | 12-05-2021 |
| | | IL 279749 A | 01-03-2021 |
| | | IL 308110 A | 01-12-2023 |
| | | IL 323052 A | 01-10-2025 |
| | | JP 7454243 B2 | 22-03-2024 |
| | | JP 7709639 B2 | 17-07-2025 |
| | | JP 2021535762 A | 23-12-2021 |
| | | JP 2024023823 A | 21-02-2024 |
| | | JP 2025126186 A | 28-08-2025 |
| | | SG 11202010437T A | 28-01-2021 |
| | | US 2021113373 A1 | 22-04-2021 |
| | | US 2021267800 A1 | 02-09-2021 |
| | | US 2022257414 A1 | 18-08-2022 |
| | | US 2024374425 A1 | 14-11-2024 |
| | | US 2024407951 A1 | 12-12-2024 |
| | | WO 2020008323 A1 | 09-01-2020 |

EPO FORM P0459

For more details about this annex : see Official Journal of the European Patent Office, No. 12/82

**REFERENCES CITED IN THE DESCRIPTION**

**Patent documents cited in the description**

- CN 113924143 A